# EUROPEAN PATENT APPLICATION

(11) **EP 1 541 588 A1**
(43) Date of publication of application: **15.06.2005**
(21) Application number: 03765324.3
(22) Date of filing: 18.07.2003
(51) Int. Cl.: C07K 16/00, C07K 19/00, C12N 15/09, G01N 33/53

(54) **SINGLE CHAIN ANTIBODY AND UTILIZATION THEREOF**

(30) Priority: 18.07.2002 JP 2002210067
(71) Applicant: CellFree Sciences Co., Ltd., Yokohama-shi, Kanagawa 230-0046 (JP)
(72) Inventor: ENDO, Yaeta, Matsuyama-shi, Ehime 791-8016 (JP); KAWASAKI, Takayasu, Iwasaki Residence 401, Matsuyama-shi, Ehime 790-0854 (JP); SAWASAKI, Tatsuya, Matsuyama-shi, Ehime 790-0811 (JP)
(74) Representative: De Clercq, Ann
(86) International application number: PCT/JP2003/009140
(87) International publication number: WO 2004/009639

(57) **Abstract**

The present invention provides a single chain antibody that retains its original specific binding activity with an antigen, and a labeled single chain antibody in which a labeling substance is bound to the single chain antibody. Specifically, the labeled single chain antibody of the present invention can be produced by linking a labeling substance to a linker part of a single chain antibody. The antibody is produced using a wheat embryo-derived cell-free protein synthesis system, and production is carried out in a low reductive state that allows an intramolecular disulfide bond to be retained. Further, bonding the antibody to a solid phase via the labeling substance enables production of an immobilized single chain antibody as well as a method for analyzing an antigen-antibody reaction using the immobilized single chain antibody.

## Description

This application claims the benefit of priority from Japanese Patent Application No. 2002-210067, the entire contents of which arc incorporated herein by reference.

### BACKGROUND OF THE INVENTION

### Field of the Invention

The present invention relates to a single chain antibody having a structure in which a heavy chain and a light chain of the antibody are crosslinked through a linker, a labeled single chain antibody in which a labeling substance is provided in a linker part of the aforementioned antibody, and methods for utilizing the same.

### Description of the Related Art

A single chain antibody is small in size in comparison to a complete IgG since it comprises only an antigen-binding region, and thus a feature thereof is that non-specific binding to a cell can be lessened. When using a single chain antibody for analysis of an antigen-antibody reaction, a method has been developed in which various labels are attached to antibodies for the purpose of tracking immunoreaction (Cloutier, S. M. et al., Mol. Immunol. , 37, 1067-1077 (2000)). Although various methods have been proposed for labeling an antibody, such as a method in which biotin or the like is bound to the C terminus or N terminus of the antibody using a biotin ligase (Cloutier, S. M. et al., Mol. Immunol., 37, 1067-1077 (2000)), a problem has existed in that the activity of the antibody to bind with an antigen is reduced by the label.

In recent years, the development of techniques for immobilizing this kind of antibody on chips or beads or the like for the purpose of detecting specific antigens present on a cell surface rapidly and in large amounts has also been remarkable (Mitchell, P., Nature Biotechnology, 20, 225-229 (2002)). More specifically, while techniques such as microspotting, microprinting, and chemical modification are used, each of these has problems that the binding activity of the antibody to an antigen is lowered, the high-density application is difficult, and the like.

Meanwhile, a method has also been proposed in which substances having specific binding ability such as streptavidin/biotin that covalently bind to immobilized protein reaction plates are bonded as linkers. However, in these methods also, no examples exist in which an immobilized antibody maintained its binding ability against the antigen.

### SUMMARY OF THE INVENTION

It is an object of this invention to provide an antibody in which a single chain antibody having a structure in which a heavy chain and a light chain of the antibody are crosslinked through a linker maintains binding activity with an antigen, a labeled single chain antibody produced by labeling the aforementioned antibody, and methods that utilize these. A further object of this invention is to provide a method for immobilizing an antibody while maintaining the binding ability of the antibody against its antigen, a labeled single chain antibody for use in the method, and a method for analyzing an antigen-antibody reaction that uses the labeled single chain antibody.

After conducting concentrated research to solve the above-described problems, the present inventors bound biotin to the linker part of a single chain antibody in which the heavy chain and the light chain of the antibody were connected through a linker, and brought the single chain antibody into contact with a reaction plate whose surface was coated with streptavidin to bind the antibody to the reaction plate. When we brought an antigen into contact with the immobilized single chain antibody produced in this manner, we found that the binding ability of the antibody against the antigen was maintained at an extremely high level. This invention was accomplished based on these findings.

More specifically, the present invention provides the following:
1. A single chain antibody comprising having a structure in which a heavy chain and a light chain of the antibody are crosslinked through a linker, or a labeled single chain antibody comprising carrying a labeling substance in the linker part of the single chain antibody.
2. A single chain antibody having a structure in which a heavy chain and a light chain of the antibody are crosslinked through a linker, or a labeled single chain antibody carrying a labeling substance in the linker part of the single chain antibody, wherein the heavy chain and the light chain of the antibody are variable regions.
3. A labeled single chain antibody having a structure in which a heavy chain and a light chain of the antibody are crosslinked through a linker, and carrying a labeling substance in the linker part, wherein the labeling substance is a substance that is capable of binding to a polypeptide of the linker part of the antibody in the presence of a specific enzyme.
4. A labeled single chain antibody having a structure in which a heavy chain and a light chain that are variable regions of the antibody are crosslinked through a linker, and carrying a labeling substance in the linker part, wherein the labeling substance is a substance that is capable of binding to a polypeptide of the linker part of the antibody in the presence of a specific enzyme.
5. A labeled single chain antibody having a structure in which a heavy chain and a light chain of the antibody are crosslinked through a linker, and carrying a labeling substance in the linker part, wherein the labeling substance is incorporated as one part of the linker part of the antibody.
6. A labeled single chain antibody having a structure in which a heavy chain and a light chain that are variable regions of the antibody are crosslinked through a linker, and carrying a labeling substance in the linker part, wherein the labeling substance is incorporated as one part of the linker part of the antibody.
7. A labeled single chain antibody having a structure in which a heavy chain and a light chain of the antibody are crosslinked through a linker, and carrying in the linker part a labeling substance that is capable of binding to a polypeptide of the linker part of the antibody in the presence of a specific enzyme, wherein the labeling substance is biotin and the enzyme is a biotin ligase.
8. A labeled single chain antibody having a structure in which a heavy chain and a light chain that are variable regions of the antibody are crosslinked through a linker, and carrying in the linker part a labeling substance that is capable of binding to a polypeptide of the linker part of the antibody in the presence of a specific enzyme, wherein the labeling substance is biotin and the enzyme is a biotin ligase.
9. The single chain antibody or labeled single chain antibody according to any one of the above 1 to 8, which has a Kd value that is equivalent to a Kd value of a naturally occurring antibody and which was produced by a cell-free protein translation system using wheat embryo.
10. A DNA, wherein DNAs encoding a heavy chain and a light chain of an antibody having binding ability against a specific antigen are linked through a DNA encoding a linker.
11. A DNA in which DNAs encoding a heavy chain and a light chain of an antibody having binding ability against a specific antigen are linked through a DNA encoding a linker, wherein the heavy chain and the light chain of the antibody are variable regions.
12. A DNA in which DNAs encoding a heavy chain and a light chain of an antibody having binding ability against a specific antigen are linked through a DNA encoding a linker, wherein the DNA encoding a linker comprises a nucleotide sequence that is capable of binding with a labeling substance in the presence of a specific enzyme after translation.
13. A DNA in which DNAs encoding a heavy chain and a light chain that are variable regions of an antibody having binding ability against a specific antigen are linked through a DNA encoding a linker, wherein the DNA encoding a linker comprises a nucleotide sequence that is capable of binding with a labeling substance in the presence of a specific enzyme after translation.
14. A DNA in which DNAs encoding a heavy chain and a light chain of an antibody having binding ability against a specific antigen are linked through a DNA encoding a linker comprising a nucleotide sequence that is capable of binding with a labeling substance in the presence of a specific enzyme after translation, wherein the nucleotide sequence that is capable of binding with a labeling substance encodes an amino acid sequence that is recognized by a biotin ligase.
15. A DNA in which DNAs encoding a heavy chain and a light chain that are variable regions of an antibody having binding ability against a specific antigen are linked through a DNA encoding a linker comprising a nucleotide sequence that is capable of binding with a labeling substance in the presence of a specific enzyme after translation, wherein the nucleotide sequence that is capable of binding with a labeling substance encodes an amino acid sequence that is recognized by a biotin ligase.
16. A method for producing a labeled single chain antibody, wherein the DNA of any of the preceding 10 to 15 is subject to transcription and translation using a protein synthesis system in the presence of a labeling substance and a specific enzyme.
17. A method for producing a single chain antibody or a labeled single chain antibody, wherein the DNA of either of the foregoing 10 or 11 is subject to transcription and translation using a protein synthesis system.
18. The method for producing a single chain antibody or a labeled single chain antibody according to the preceding 16 or 17, wherein the protein synthesis system is a wheat embryo-derived cell-free protein translation system, and a concentration of a reducing agent in a translation reaction solution thereof is a concentration at which a disulfide bond of a single chain antibody to be produced is maintained and cell-free protein synthesis is enabled.
19. The method for producing a single chain antibody or a labeled single chain antibody according to the preceding 18, wherein the method is conducted in the presence of an enzyme that catalyzes a disulfide bond exchange reaction.
20. A single chain antibody or a labeled single chain antibody having a Kd value that is equivalent to a Kd value of a naturally occurring antibody, wherein the single chain antibody or the labeled single chain antibody is produced by the method for producing a single chain antibody or labeled single chain antibody according to the preceding 19 using a wheat embryo-derived cell-free protein translation system.
21. A method for producing an immobilized single chain antibody, wherein any one of the antibodies described hereunder is contacted with a reaction plate compartmentalized into a plurality of regions having on the surface thereof a substance that binds specifically with a labeling substance of the antibody:
   (1) a labeled single chain antibody, wherein the antibody has a structure in which a heavy chain and a light chain of the antibody are crosslinked through a linker and the antibody carries a labeling substance in the linker part;
   (2) a labeled single chain antibody having a structure in which a heavy chain and a light chain of the antibody are crosslinked through a linker, and carrying a labeling substance in the linker part, wherein the heavy chain and the light chain of the antibody are variable regions;
   (3) a labeled single chain antibody having a structure in which a heavy chain and a light chain of the antibody are crosslinked through a linker, and carrying a labeling substance in the linker part, wherein the labeling substance is a substance that is capable of binding to a polypeptide of the linker part of the antibody in the presence of a specific enzyme;
   (4) a labeled single chain antibody having a structure in which a heavy chain and a light chain that are variable regions of the antibody are crosslinked through a linker, and carrying a labeling substance in a linker part, wherein the labeling substance is a substance that is capable of binding to a polypeptide of the linker part of the antibody in the presence of a specific enzyme;
   (5) a labeled single chain antibody having a structure in which a heavy chain and a light chain of the antibody are crosslinked through a linker, and carrying a labeling substance in the linker part, wherein the labeling substance is incorporated as one part of the linker part of the antibody;
   (6) a labeled single chain antibody having a structure in which a heavy chain and a light chain that are variable regions of the antibody are crosslinked through a linker, and carrying a labeling substance in the linker part, wherein the labeling substance is incorporated as one part of the linker part of the antibody;
   (7) a labeled single chain antibody having a structure in which a heavy chain and a light chain of the antibody are crosslinked through a linker, and carrying in the linker part a labeling substance that is capable of binding to a polypeptide of the linker part of the antibody in the presence of a specific enzyme, wherein the labeling substance is biotin and the enzyme is a biotin ligase;
   (8) a labeled single chain antibody having a structure in which a heavy chain and a light chain that are variable regions of the antibody are crosslinked through a linker, and carrying in a linker part a labeling substance that is capable of binding to a polypeptide of the linker part of the antibody in the presence of a specific enzyme, wherein the labeling substance is biotin and the enzyme is a biotin ligase.
22. A method for producing an immobilized single chain antibody according to the method described in the preceding 21, wherein two or more kinds of different immobilized single chain antibodies are immobilized on a reaction plate compartmentalized into a plurality of regions.
23. The production method according to the preceding 21 or 22, wherein a labeling substance is biotin and a substance that binds specifically with the labeling substance is streptavidin.
24. An immobilized single chain antibody prepared by the production method according to any one of the preceding 21 to 23.
25. A method for analyzing an antigen-antibody reaction, wherein a test substance is contacted with the immobilized single chain antibody according to the preceding 24, and binding ability of the test substance against the immobilized single chain antibody is analyzed.
26. A method for analyzing an antigen-antibody reaction, comprising the steps of:
   (1) preparing a labeled single chain antibody under conditions in which a disulfide bond of a single chain antibody is retained, comprising the step of the following (i) or (ii):
      (i) producing a labeled single chain antibody by subjecting a DNA, in which DNAs encoding a heavy chain and a light chain of an antibody having binding ability with a specific antigen are linked through a DNA encoding a linker comprising a nucleotide sequence that is capable of binding with a labeling substance in the presence of a specific enzyme after translation, to transcription and translation using a wheat cell-free protein synthesis system in the presence of a specific enzyme; or
      (ii) producing a labeled single chain antibody by subjecting a DNA, in which DNAs encoding a heavy chain and a light chain that are variable regions of an antibody having binding ability with a specific antigen are linked through a DNA encoding a linker comprising a nucleotide sequence that is capable of binding with a labeling substance in the presence of a specific enzyme after translation, to transcription and translation using a wheat cell-free protein synthesis system in the presence of a specific enzyme;
   (2) preparing a substance (adapter substance) that binds specifically with a labeling substance of a labeled single chain antibody in a case where the labeling substance of the labeled single chain antibody is an immobilizing substance, comprising the steps of:
      (i) immobilizing a substance (adapter substance) that binds specifically with a labeling substance of a labeled single chain antibody on a reaction plate compartmentalized into a plurality of regions;
      (ii) removing the substance (adapter substance) that binds specifically with a labeling substance of a labeled single chain antibody that was not immobilized to the reaction plate in the preceding (i); and
      (iii) before and after the step of the preceding (i) or (ii), removing nonspecific adsorption from the reaction plate as appropriate;
   (3) preparing an immobilized labeled single chain antibody in a case where a labeling substance of the labeled single chain antibody is an immobilizing substance, comprising the steps of:
      (i) adding a required amount of the labeling substance of the labeled single chain antibody prepared in (i) or (ii) of the preceding (1) onto a reaction plate compartmentalized into a plurality of regions having a substance (adapter substance) of (2) that binds specifically with the labeling substance of the labeled single chain antibody on the surface thereof, whereby to contact;
      (ii) removing a labeled single chain antibody that was not immobilized to the substance (adapter substance) that binds specifically to the labeled single chain antibody on the reaction plate in the preceding (i); and
      (iii) following the preceding step (ii), removing nonspecific adsorption from the reaction plate as appropriate;
   (4) preparing a labeled single chain antibody in a case where a labeling substance is a signal substance, comprising the steps of:
      (i) removing nonspecific adsorption from a reaction plate compartmentalized into a plurality of regions as appropriate; and
      (ii) adding a required amount of the labeling substance of the labeled single chain antibody prepared in (i) or (ii) of the above (1) to the reaction plate;
   (5) adding a required amount of a test substance to a reaction plate according to the above (3) or (4), and analyzing the binding ability of a labeled single chain antibody with the test substance; and
   (6) based on the binding ability result obtained in the preceding (5), qualitatively or quantitatively determining the interaction between the labeled single chain antibody and the test substance.
27. A reagent kit for measuring an antigen-antibody reaction, comprising a reagent to be used in the analysis method according to the preceding 25 or 26.

### BRIEF DESCRIPTION OF THE DRAWINGS

FIG. 1 is a view showing the structure of a translation template of the single chain antibody of this invention.
FIG. 2 is a photograph of electrophoresis showing the degree of binding of biotin to a single chain antibody caused by a biotin ligase.
FIG. 3 is a view showing the degree of specific binding to an antigen of the labeled single chain antibody of this invention
FIG. 4 is a view showing a curve of association and dissociation of the labeled single chain antibody of this invention and an antigen.
FIG. 5 is a view showing the degree of binding between an antigen and a single chain antibody in which biotin was bonded in an area other than a linker part.
FIG. 6 is a view showing the degree of binding to a nickel column of a single chain antibody having a polyhistidine peptide in a linker part.

### DETAILED DESCRIPTION OF THE INVENTION

### (1) Single chain antibody and labeled single chain antibody

A single chain antibody used in this invention may be any kind of substance, as long as it is a substance in which a heavy chain and a light chain of an antibody are connected through a linker and which has activity for binding with an antigen for which the antibody has specific binding affinity. Preferably, the substance used is one in which the heavy chain of an antibody is positioned at the N terminus of the single chain antibody molecule. As an antibody, a monoclonal antibody having activity that recognizes and binds with a specific antigen is preferable. Further, with respect to a heavy chain and light chain of an antibody, it is not necessary that the substance comprise the full length thereof, as long as the substance comprises a part that is sufficient for recognizing an antigen and for having specific binding affinity thereto. More specifically, a variable region is preferably used.

A linker is not particularly limited, as long as it has a length that is sufficient for a heavy chain and a light chain of an antibody to be crosslinked through the linker, and also has a structure for having a labeling substance. In general, a polypeptide comprising 10 to 30 amino acids is preferably used. A specific structure can be suitably selected in accordance with a labeling substance that is described hereunder.

As a labeling substance, a substance that can be used for the purpose of labelling the single chain antibody of this invention (hereunder, this is sometimes referred to as "signal substance") and a substance that can be used for the purpose of immobilizing the single chain antibody of this invention (hereunder, this is sometimes referred to as "immobilizing substance") are preferable. More specifically, examples of a signal substance include a fluorescent dye that is capable of binding to an amino acid, such as a dye belonging to fluorescein, rhodamine, eosin, or NBD; a photosensitizer, such as methylene blue or rose bengal; or a substance that imparts a specific signal in nuclear magnetic resonance (NMR), for example an amino acid comprising a fluorine or phosphorus atom. As an immobilizing substance (hereunder, this is sometimes referred to as "adapter substance"), any substance may be used as long as it is a substance that binds with a specific substance that has been bound to a solid phase surface. Examples of a combination of an immobilizing substance and an adapter substance include various types of receptor proteins and a ligand thereof, such as biotin and a biotin-binding protein such as avidin or streptavidin; maltose and a maltose-binding protein; guanine nucleotide and G protein; a polyhistidine peptide and a metal ion such as nickel or cobalt; glutathione-S-transferase and glutathione; a DNA-binding protein and a DNA; an antibody and an antigen molecule (epitope); calmodulin and a calmodulin-binding peptide; ATP-binding protein and ATP; or estradiol receptor protein and estradiol. Either of these substances may the immobilizing substance or the adapter substance. Among them, preferably biotin is used as an immobilizing substance and streptavidin as an adapter substance, or a polyhistidine peptide is used as an immobilizing substance and nickel or the like is used as an adapter substance.

A substance that is capable of binding to a polypeptide of a linker part of an antibody in the presence of a specific enzyme in a method for binding a substance to the linker part can also be used as a labeling substance. Examples of this type of substance include biotin and the like. When using biotin as a labeling substance, examples of a specific enzyme include a biotin ligase, and examples of a linker include a substance having an amino acid sequence that can be recognized by a biotin ligase.

Further, a labeling substance may be a substance that is incorporated as one part of a linker part of an antibody, and as a specific example thereof a polyhistidine peptide may be mentioned. In this case, a substance comprising a polyhistidine peptide may be used as a linker.

Binding of a labeling substance to a linker part, or incorporation a labeling substance therein, can be carried out according to a known method in accordance with the signal substance to be used or the properties of the immobilizing substance and adapter substance.

### (2) Method for producing single chain antibody and labeled single chain antibody

A single chain antibody and labeled single chain antibody of this invention can be produced, for example, according to the methods described below. First, (i) a monoclonal antibody that recognizes a protein of interest or a part thereof as an antigen is prepared, and (ii) DNA encoding the monoclonal antibody is acquired. Then, the sequences encoding the heavy chain and light chain thereof are identified, and these are linked together sandwiching a nucleotide sequence encoding the linker (hereunder, this DNA fragment may sometimes be referred to as "single chain antibody unit"). (iii) The protein that is encoded by the thus-produced single chain antibody unit is then synthesized by a suitable method that properly maintains the structure thereof. In the case of binding a labeling substance to the linker part at the time of synthesis or after synthesis, the appropriate binding procedure is conducted. These methods are described in detail hereunder.

### (i) Preparation of monoclonal antibody

An antigen of the single chain antibody of this invention is not particularly limited, and may be any substance as long as the substance has immunogenicity. More specifically, for example, a sugar chain of Salmonella or the like may be mentioned. A known method conventionally used in the art can be used as a method of preparing a monoclonal antibody that specifically recognizes these antigens, and for a polypeptide used as an antigen, a sequence that is suitable as an epitope (antigenic determinant) with high antigenicity can be selected in accordance with a known method and used. As a method of selecting an epitope, for example, commercially available software such as Epitope Adviser (manufactured by Fujitsu Kyushu System Engineering) or the like can be used.

As a polypeptide used as the aforementioned antigen, a synthetic peptide that was synthesized in accordance with a known method is preferably used. Although a polypeptide to be used as an antigen may be prepared in an appropriate solution or the like in accordance with a known method and then used to immunize a mammal such as rabbit or mouse, in order to conduct stable immunization and raise the antibody titer, immunization is preferably conducted using an antigen peptide that forms a conjugate with a suitable carrier protein, with the addition of an adjuvant or the like.

The route of administration of an antigen at the time of immunization is not particularly limited, and for example, a subcutaneous, intraperitoneal, intravenous or intramuscular route may be used. More specifically, for example, a method may be used in which BALB/c mice are inoculated with an antigen polypeptide several times at intervals of several days to several weeks. Regarding intake of the antigen, while an intake of from 0.3 to 0.5 mg/per inoculation is preferable when the antigen is a polypeptide, the intake can be appropriately adjusted in accordance with the kind of polypeptide and the species of animal to be immunized.

After immunization, blood is tentatively collected as appropriate to verify an increase in antibody titer by a method such as enzyme-linked immunosorbent assay (hereunder, this is sometimes referred to as "ELISA") or Western blotting, and blood is then collected from an animal in which the antibody titer has increased sufficiently. By subjecting the obtained blood to a suitable process used in preparation of an antibody, a polyclonal antibody can be obtained. More specifically, for example, a method may be mentioned in which purified antibody is acquired by purifying the antibody component from serum in accordance with a known method. A monoclonal antibody can also be produced using a hybridoma produced by fusing myeloma cell and spleen cell of the animal in accordance with a known method (Milstein et al., Nature, 256, 495 (1975)). A monoclonal antibody can be acquired, for example, by the method described below.

First, antibody-forming cells are acquired from the aforementioned animal in which the antibody titer was raised by immunization of an antigen. Antibody-forming cells are the plasma cells and the precursor cells thereof, lymphoid cells, and while they may be acquired from any part of the individual, they are preferably acquired from the spleen, lymph node, peripheral blood or the like. As myeloma cells to be fused with these cells, in general, an established cell line acquired from mouse, such as 8-azaguanine resistant mouse (derived from BALB/c or the like) myeloma cell line P3X63-Ag 8.653 (ATCC: CRL-1580) or P3-NS1/1Ag 4.1 (Riken Cell Bank: RCB0095) or the like are preferably used. Fusion of the cells can be carried out by mixing the antibody-forming cells and myeloma cells at an appropriate ratio using a suitable cell fusion medium, for example, RPMI 1640 or Iscove's modification of Dulbecco's medium (IMDM), or a medium in which 50% polyethyleneglycol is dissolved in Dulbecco's modified Eagle's medium (DMEM) or the like. Fusion of cells can also be conducted by an electrofusion method (U. Zimmermann et al., Naturwissenschaften, 68, 577 (1981)).

A hybridoma can be selected by utilizing the fact that the myeloma cell line used is an 8-azaguanine resistant line and culturing for an appropriate time at 37 °C with 5% CO₂ in a normal culture medium containing a suitable amount of hypoxanthine amino-pterin thymidine (HAT) solution (HAT culture medium). The selection method can be suitably selected and used in accordance with the myeloma cell line used. A monoclonal antibody can be obtained by analyzing according to the aforementioned method antibody titers of antibodies produced by selected hybridomas, isolating a hybridoma producing an antibody having a high antibody titer by a limiting dilution method or the like, and purifying the monoclonal antibody from culture supernatant obtained by culturing the isolated fused cell in a suitable medium, by an appropriate method such as ammonium sulfate fractionation or affinity chromatography. A commercially available monoclonal antibody purification kit can also be used to purify the monoclonal antibody. Further, peritoneal fluid containing a large quantity of the monoclonal antibody of this invention can also be obtained by allowing the antibody-producing hybridoma obtained in the manner described above to proliferate intraperitoneally in an animal of the same family as the immunized animal or in nude mice or the like.

### (ii) Acquisition of DNAs encoding heavy chain and light chain of monoclonal antibody and preparation of single chain antibody unit

As a specific method for acquiring DNAs encoding the heavy chain (H chain) and the light chain (L chain) of the monoclonal antibody acquired in the above (i), a method may be mentioned in which the amino acid sequences of one part of the L chain and H chain of immunoglobulin obtained from a hybridoma producing the monoclonal antibody, preferably, parts of the amino acid sequences having a variable region (V region), are analyzed, and on the basis of the amino acid sequences the gene encoding it is cloned. Here, a variable region of the L chain and the H chain of the monoclonal antibody is preferably a region comprising a framework region (FR) and a hypervariable region (CDR).

As DNA encoding a variable region of a H chain and L chain obtained in this manner, for example, DNA comprising the sequence described in Anand, N. N., et al., J. Biol. Chem., 266, 21874-21879 (1991) and the like may be mentioned as DNA of a single chain antibody that recognizes O-antigen of Salmonella.

A single chain antibody unit can be prepared by sandwiching DNA encoding a linker between the thus-obtained DNAs encoding the variable regions of the H chain and L chain, and connecting the two DNA fragments by an appropriate method. In this case, it is not necessary to obtain the single chain antibody unit separately as a DNA fragment, and it may be constructed at the same time as insertion into an expression vector or the like as described later. As DNA encoding a linker, any substance may be used as long as it is DNA encoding a linker as described in (1). More specifically, for example, DNA encoding a linker that includes an amino acid sequence that is recognized by a biotin ligase (Peter J. Schatz (1993), Biotechnology, 11 (1138-1143)) is preferable, and examples thereof include the substance represented by SEQ ID NO: 1. Further, as an example in which a labeling substance is incorporated as one part of the linker part, a substance comprising a nucleotide sequence encoding a polyhistidine peptide or the like may be mentioned.

DNA encoding a linker can be produced using a method used conventionally, and the DNA is preferably produced by chemical synthesis.

### (iii) Production of single chain antibody

A single chain antibody can be produced by connecting the thus-obtained single chain antibody unit to a suitable promoter to be under the control thereof, and introducing this into a host, or by conducting transcription by an appropriate method and then expressing the single chain antibody under conditions which retain a disulfide bond of the single chain antibody to be produced using a cell-free protein translation system. An antibody having low binding ability against an antigen can also be acquired as an antibody having higher binding ability by use of a known evolutionary engineering technique.

A suitable promoter can be appropriately selected in accordance with a host to be used or the RNA synthetase used in transcription. More specifically, when using SP6 RNA synthetase for transcription, SP6 promoter is preferably used. In the cell-free protein translation system, a base sequence that augments translational activity is preferably inserted between the promoter and the single chain antibody unit. Specific examples of known base sequences that augment translational activity include the 5'-cap structure (Shatkin, Cell, 9, 645- (1976)), Kozak sequence (Kizak, Nucleic Acid Res., 12, 857- (1984)) and the like in eucaryotes, and Shine-Dalgarno sequence and the like in prokaryotes. Further, it has been found that translation promoting activity is also present in the 5'-nontranslated leader sequences of RNA virus (Japanese Patent No. 2814433), and a method has been developed which efficiently conducts protein synthesis using these sequences (Japanese Patent Laid-Open No. 10-146197). In addition, with respect to a random sequence, a sequence obtained by a method that selects a translation enhancer sequence by taking influence on polysome formation as an indicator may also be mentioned (specification of Japanese Patent Application No. 2001-396941). Hereunder, DNA produced in this manner may sometimes be referred to as "translation template."

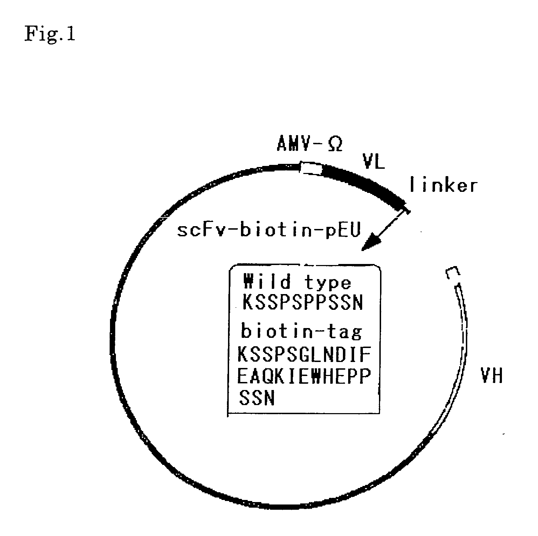

As a specific example of a translation template, a substance having the structure shown in FIG. 1 may be mentioned as an example of a substance recognizing O-antigen of Salmonella.

As a host into which a translation template is introduced, a wheat embryo-derived cell-free protein synthesis system that can be used in normal protein synthesis and which is capable of retaining a disulfide bond of a single chain antibody is used. The reason this system is used is that an antibody produced with a different cell-free protein synthesis system is unable to sufficiently retain a tertiary structure for recognizing an antigen, and exhibits only a low Kd value (Alexander Zdanov et al., Proc. Natl. Acad. Sci. USA, Vol 91, pp. 6423-6427 (1994); C. Roger Mackenzie et al., The Journal of Biological Chemistry, Vol. 271, pp. 1527-1533 (1998)). As a specific example of cell extract from wheat embryo to be used in this invention, the commercially available Proteios™ (manufactured by Toyobo Co., Ltd.) or the like may be mentioned.

Further, a reaction solution which can retain an intramolecular disulfide bond and also synthesize a protein can be prepared by adjusting the concentration of a reducing agent among the ingredients necessary for protein synthesis of a reaction solution of the above wheat embryo-derived cell-free translation system (hereunder, this is sometimes referred to as "weak reductive translation reaction solution"). Examples of a specific reducing agent and the concentration thereof include dithiothreitol (hereunder, sometimes referred to as "DTT") at a final concentration of 20 to 70 µM, preferably 30 to 50 µM, 2-mercaptoethanol at a final concentration of 0.1 to 0.2 mM, and glutathione/oxidized glutathione at a final concentration within a range of 30 to 50µM/l to 5 µM.

The concentration of a reducing agent in the translation reaction solution is not limited to the above concentrations, and may be suitably modified in accordance with the protein to be synthesized. While a method for selecting the range of optimal concentration of a reducing agent is not particularly limited, for example, a method in which assessment is made based on the effect of an enzyme catalyzing a disulfide bond exchange reaction may be mentioned. More specifically, translation reaction solutions in which the concentration of a reducing agent is variously adjusted are prepared, and an enzyme that catalyzes a disulfide bond exchange reaction is added to these solutions, to conduct synthesis of a protein having an intramolecular disulfide bond. As a control experiment, protein synthesis is carried out in a similar manner in the same translation reaction solutions without adding the enzyme that catalyzes a disulfide bond exchange reaction. A solubilized component of the protein synthesized in the above manner is then isolated by, for example, a method such as centrifugation. A reaction solution in which the solubilized component constitutes 50% (solubilization ratio 50%) or more of the total volume and in which the solubilized component increased after addition of the enzyme that catalyzes a disulfide bond exchange reaction can be judged as suitable as a reaction solution that conducts synthesis while retaining an intramolecular disulfide bond of the protein in its original state. Further, of the ranges of concentration of a reducing agent that was selected on the basis of the aforementioned effect of an enzyme that catalyzes a disulfide bond exchange reaction, concentrations of a reducing agent that generate the largest amount of synthesized protein can be selected as a further preferable concentration range.

Methods that can be used to prepare a reaction solution having the aforementioned reducing agent concentration include a method in which cell extract for wheat embryo-derived cell-free protein synthesis that does not include a reducing agent is prepared, and then ingredients required for a wheat embryo-derived cell-free protein translation system are added thereto together with a reducing agent at a concentration within the above concentration range, and a method in which a reducing agent is removed from cell extract for wheat embryo-derived cell-free protein synthesis such that the concentration of the reducing agent is within the aforementioned concentration range. Since cell extract for wheat embryo-derived cell-free protein synthesis requires advanced reducing conditions when extracting, a method in which a reducing agent is removed from this solution after extraction is more convenient. As a method for removing a reducing agent from cell extract, a method using a carrier for gel filtration and the like may be mentioned. More specifically, for example, a method in which a Sephadex G-25 column is previously equilibrated with a suitable buffer solution that does not include a reducing agent, and cell extract is then passed therethrough may be mentioned.

Further, the cell extract may also be used after forming the cell extract into a lyophilized product by lyophilizing, and adding a suitable buffer solution thereto. Preferably the total concentration of a deliquescent substance is made 60 mM or less when lyophilizing. Lyophillization can also be conducted after adding the aforementioned translation template to the cell extract.

Further, for a substance exhibiting deliquescence (deliquescent substance) in the aforementioned lyophilized product, a content that does not lower storage stability in a lyophilized condition is preferably 0.01 parts by weight or less relative to 1 part by weight of a protein contained in the lyophilized product, and particularly preferably the content is 0.005 parts by weight or less relative thereto. In this connection, the weight of a protein mentioned here refers to a weight calculated by measurement of absorbance (260, 280, 320 nm).

Hereunder, cell extract in which the concentration of a reducing agent was adjusted as described above is sometimes referred to as "weak reductive translation reaction solution."

Further, by carrying out a translation reaction in which an enzyme that catalyzes a disulfide bond exchange reaction is further added to a weak reductive translation reaction solution, it is possible to conduct highly efficient synthesis of a protein that retains an intramolecular disulfide bond. As an enzyme that catalyzes a disulfide bond exchange reaction, for example, protein disulfide isomerase or the like may be mentioned. The amount of these enzymes to be added to a wheat embryo-derived cell-free translation system can be suitably selected in accordance with the kind of enzyme. More specifically, when adding protein disulfide isomerase to a translation reaction solution that is cell extract for cell-free protein synthesis extracted from wheat embryo, which contains as a reducing agent 20 to 70 µM of DTT, and preferably 30 to 50 µM thereof, the protein disulfide isomerase is added to bring to a final concentration within the range of 0.01 to 10 µM, and preferably 0.5 µM. With respect to the stage for adding an enzyme, from the viewpoint of efficiency of disulfide bond formation, the enzyme is preferably added prior to the start of the cell-free translation reaction.

Examples of cell-free protein translation systems derived from seed of plants other than wheat include those derived from gramineous plants such as barley, rice and corn. However, of these cell-free protein translation systems, use of wheat embryo extract is particularly preferable, and a method for producing a single chain antibody will be explained in detail below taking as an example a case using this cell extract.

As a method for selecting wheat embryo, for example, the method of Johnston, F. B. , et al., Nature, 179, 160-161 (1957) can be used, and as a method for producing cell extract from the embryo, a method described in Erickson, A. H., et al. Meth. In Enzymol., 96, 38-50 (1996) or the like can be used.

According to a preparation method advantageously utilized in this invention, wheat embryo extract can be obtained by collecting wheat embryo extract and purifying the extract by gel filtration or the like. Gel filtration can be conducted, for example, using a gel filtration device such as a Sephadex G-25 column. The compositions and concentrations of the various components in a gel filtration solution are known in the art, and those used in a method for producing wheat embryo extract for cell-free protein synthesis may be adopted. With respect to a solution for equilibrating a Sephadex G-25 column, by using a solution that does not contain a reducing agent, more specifically, for example, a solution containing HEPES-KOH, potassium acetate, magnesium acetate, or L-form amino acids, approximately 97% of a reducing agent contained in the extract can be absorbed. Specifically, when extraction is conducted using extract from wheat embryo containing 1 mM of DTT as a reducing agent, it is possible to ultimately obtain wheat embryo extract containing approximately 30 µM of DTT. However, because the activity of wheat embryo extract in which the concentration of a reducing agent has been lowered is noticeably reduced by cryopreservation, a step of removing a reducing agent is preferably conducted immediately prior to a translation reaction in which the extract is to be used.

Microorganisms, in particular spores such as filamentous bacteria (mold) may sometimes be contained in the embryo extract after gel filtration, and these microorganisms are preferably removed. Since proliferation of microorganisms is observed, in particular, in long-term (1 day or more) cell-free protein synthesis reaction, the prevention thereof is important. Although a technique for removing microorganisms is not particularly limited, the use of a filtration sterilization filter is preferable. The pore size of a filter is not particularly limited as long as the filter is capable of removing microorganisms that may contaminate the extract, and normally a pore size of 0.1 to 1 micrometer, preferably 0.2 to 0.5 micrometers, is adequate. In this connection, since the size of small categories of spores of *Bacillus subtilis* is 0.5 µm × 1 µm, the use of a 0.20 micrometer filter (for example, Minisart™, manufactured by Sartorius HPLC) is also effective for removing spores. When filtering, preferably, filtering is first conducted using a filter with a large pore size, and then filtering is conducted using a filter with a pore size that is capable of removing microorganisms that may be contained in the initial filtrate.

Cell extract obtained in this manner is purified to the extent that endosperm containing a substance that inhibits a protein synthesis function (a substance such as tritin, thionin or a ribonuclease that acts on mRNA, tRNA, a translation protein factor or ribosome or the like and inhibits the function thereof) retained by or contained by the source cell itself is almost completely removed. Here, the term "purified to the extent that endosperm is almost completely removed" refers to wheat embryo extract in which an endosperm part is removed to the extent that ribosome is not substantially deadenylated, and the term "extent that ribosome is not substantially deadenylated" refers to the deadenylation rate of ribosome being less than 7%, and preferably 1% or less.

Further, since this type of cell extract from which an endosperm component has been removed contains low-molecular protein synthesis inhibitors (hereunder, these may be referred to as "low-molecular synthesis inhibitors"), preferably these low-molecular synthesis inhibitors are removed by fractionation from the components of the cell extract utilizing difference in molecular weight. It is sufficient that the molecular weight of substances to be removed (low-molecular inhibitors) be lower than that of factors necessary for protein synthesis contained in the cell extract. More specifically, substances having a molecular weight of 50,000 to 14,000 or less, and preferably 14,000 or less may be mentioned.

As a method for removing low-molecular synthesis inhibitors from cell extract, a known method conventionally used in the art can be used, and as a specific example thereof a method using dialysis through a dialysis membrane, gel filtration, or ultrafiltration may be mentioned. Of these, a method using dialysis (dialysis method) is preferable from the viewpoint of ease of supply of the substance to an internal dialysis solution. Hereunder, an example when using a dialysis method is described in detail.

As a dialysis membrane to be used in the dialysis, a membrane with a molecular weight cutoff of 50,000 to 12,000 may be mentioned, and more specifically, a regenerated cellulose membrane with a molecular weight cutoff of 12,000 to 14,000 (manufactured by Viskase Sales, Chicago) or SpectraPor 6 with a molecular weight cutoff of 50,000 (manufactured by Spectrum Laboratories Inc., Ca., USA) or the like may be used. Dialysis is conducted according to a conventional method by introducing a suitable volume of the above-described cell extract into this type of dialysis membrane. A time period for conducting dialysis is preferably between around 30 minutes and 24 hours.

In a case where an insoluble component is generated in cell extract when removing low-molecular synthesis inhibitors, it is possible to enhance the protein-synthesizing activity of the ultimately obtained cell extract (hereunder, this is sometimes referred to as "post-treatment cell extract") by inhibiting the generation of this insoluble component (hereunder, this is sometimes referred to as "stabilization of cell extract"). As a specific method for stabilizing the cell extract, a method in which removal of the aforementioned low-molecular inhibitors is conducted in a solution containing at least a high-energy phosphate compound such as ATP or GTP may be mentioned. As a high-energy phosphate compound, ATP is preferably used. Removal is preferably conducted in a solution containing ATP and GTP, and further preferably, ATP, GTP and 20 kinds of amino acid.

When conducting removal of low-molecular inhibitors in a solution containing these components (hereunder, sometimes referred to as "stabilization components"), the step of removing the low-molecular inhibitors may be conducted after previously adding the stabilization components to the cell extract and incubating the solution. When using a dialysis method to remove low-molecular synthesis inhibitors, removal of the low-molecular inhibitors can be conducted by adding stabilization components to not only the cell extract but also to an external dialysis solution to conduct dialysis. Adding stabilization components to the external dialysis solution enables the constant supply of new stabilization components even though stabilization components are degraded during dialysis, and is thus more preferable. This technique can also be applied when using gel filtration or ultrafiltration, in which case a similar effect can be obtained by first equilibrating the respective carriers using a buffer for filtration containing stabilization components, and then providing the cell extract containing stabilization components for filtration and conducting the filtration while adding the aforementioned buffer.

The added amount of stabilization components and the time for stabilization treatment can be suitably selected in accordance with the type of cell extract and method of preparation. As an example of a method for selecting these, a method may be mentioned in which tentative amounts and kinds of stabilization components are added to cell extracts, a step of removing low-molecular inhibitors is then conducted after an appropriate period, the obtained post-treatment cell extracts are separated into solubilized components and insolubilized components by a method such as centrifugation, and of these the cell extract which has less insolubilized components is selected. Another preferable method is one in which cell-free protein synthesis is conducted using the obtained post-treatment cell extracts, and of these the cell extract with the highest protein-synthesizing activity is selected. When using cell extract with a dialysis method in the above selection methods, a method may be mentioned in which suitable stabilization components are also added to external dialysis solutions, and after conducting dialysis for an appropriate period using these, selection is conducted based on the amount of insoluble substances in the obtained cell extracts, the protein-synthesizing activity of the obtained cell extracts, or the like.

As a specific example of stabilization conditions for cell extract selected in this manner, for a case of conducting a step of removing low-molecular inhibitors by a dialysis method with the aforementioned prepared wheat embryo extract, a method may be mentioned in which 100 µM to 0.5 mM of ATP, 25 µM to 1 mM of GTP, and 25 µM to 5 mM each of 20 kinds of L-form amino acids are added to the external dialysis solution and wheat embryo extract and dialysis is conducted for 30 minutes to 1 hour or more. Dialysis may be conducted at any temperature, as long as the temperature is one at which protein synthesizing activity is not lost and dialysis is possible. More specifically, the lowest temperature is one at which the solution does not freeze, and this is normally -10 °C, preferably -5 °C, and the highest temperature is 40 °C, which is the limit for a temperature that does not impart an adverse effect on a solution used in dialysis, and 38 °C is preferable.

A method for adding stabilization components to cell extract is not particularly limited, and a method may be employed in which stabilization components are added to cell extract prior to a step of removing low-molecular inhibitors, the resulting mixture is incubated for a suitable period to undergo stabilization, and then a step of removing low-molecular synthesis inhibitors is performed, or a method may be employed in which a step of removing low-molecular synthesis inhibitors is performed using cell extract to which stabilization components were added and/or buffer solution for use in the removal step to which stabilization components were added.

Protein synthesis can be performed by preparing the aforementioned cell extract for cell-free protein synthesis with a concentration of a reducing agent that is within the above-described ranges, adding thereto, as necessary, an energy source or amino acids, translation template or tRNA or the like that are required for cell-free protein synthesis, as well as an enzyme that catalyzes a disulfide bond exchange reaction, and introducing the resulting mixture into respectively selected known systems or apparatuses. Examples of a system or apparatus for protein synthesis include a batch method (Pratt, J. M., et al., Transcription and Translation, 179-209, Hames, B. D. & Higgins, S. J., Eds., IRL Press, Oxford (1984)), a continuous cell-free protein synthesis system that continuously supplies amino acids, energy sources and the like to a reaction system (Spirin, A. S., et al., Science, 242, 1162-1164 (1988)), a dialysis method (Kigawa, et al., 21st Annual Meeting of the Molecular Biology Society of Japan, WID 6), and a overlay method (Sawasaki, T., et al., FEBS Let., 514, 102-105 (2002)).

In addition, a method which supplies template RNA, amino acids, an energy source or the like to a synthesis reaction system when required and which removes a synthesized product or degradation product at a required time (Japanese Patent Laid-Open No. 2000-333673; hereunder, this is sometimes referred to as "discontinuous gel filtration method") or the like can be used.

Among these, while use of a system that supplies amino acids or an energy source continuously or discontinuously allows a reaction to be maintained for a long period and thereby enables greater efficiency, use of a batch method is preferable when conducting protein synthesis using a weak reductive translation reaction solution, as the protein synthesis efficiency tends to be high. Further, when preparing wheat embryo extract by the method described above, the addition of tRNA is normally not necessary, as a sufficient amount of tRNA is already contained therein.

When conducting protein synthesis by a batch method, the protein can be synthesized, for example, by preincubating a synthesis reaction solution from which a translation template has been excluded for a suitable period as necessary, and then adding the translation template and incubating. As a synthesis reaction solution, for example, a solution that contains 10 to 50 mM of HEPES-KOH (pH 7.8), 55 to 120 mM of potassium acetate, 1 to 5 mM of magnesium acetate, 0.1 to 0.6 mM of spermidine, L-form amino acids (0.025 to 1 mM each), 20 to 70 µM, preferably 30 to 50 µM of DTT, 1 to 1.5 mM of ATP, 0.2 to 0.5 mM of GTP, 10 to 20 mM of creatine phosphate, 0.5 to 1.0 U/µl of RNase inhibitor, 0.01 to 10 µM of protein disulfide isomerase and 24 to 75% of wheat embryo extract can be used as a translation reaction solution.

When using this kind of translation reaction solution, preincubation is conducted at 10 to 40 °C for 5 to 10 minutes, and incubation is similarly conducted at 10 to 40 °C, preferably, 18 to 30 °C, and further preferably at 20 to 26 °C. A reaction time is the time until reaction stops, and in the batch method this is normally from about 10 minutes to 7 hours (see Pratt, J. M. , et al., Transcription and Translation, 179-209, Hames, B. D. & Higgins, S. J., Eds., IRL Press, Oxford (1984)).

When conducting protein synthesis by a dialysis method, protein synthesis is conducted using an apparatus that conducts separation by means of an external dialysis solution and a dialysis membrane that allows mass transfer, employing the synthesis reaction solution as the internal dialysis solution (see Kigawa, et al., 21st Annual Meeting of the Molecular Biology Society of Japan, WID 6).

When conducting protein synthesis using the overlay method, protein synthesis is conducted by inserting the synthesis reaction solution into a suitable container and then overlaying the external dialysis solution as described in the above dialysis method on the reaction solution in a manner that does not disturb the interface therebetween (see Sawasaki, T., et al., FEBS Let., 514, 102-105 (2002); International Patent Publication No. WO 02/24939 A1).

When conducting protein synthesis using the discontinuous gel filtration method, protein synthesis is performed by carrying out synthesis reaction using a synthesis reaction solution, and when the synthesis reaction stops, supplying template RNA, amino acids, an energy source and the like thereto, and removing a synthesized product or degradation product. More specifically, for example, after preincubating as necessary the aforementioned synthesis reaction solution from which a translation template has been excluded for an appropriate time, a translation template is added thereto and the solution is inserted into a suitable container to undergo reaction. Examples of a container include a microplate or the like. Under this reaction, for example, in the case of a reaction solution containing 48% part by volume of wheat embryo extract relative to the total volume, the synthesis reaction will stop completely after 1 hour of reaction. This can be confirmed by polyribosome analysis (Proc. Natl. Acad. Sci. USA, 97, 559-564 (2000)) using sucrose density gradient centrifugation or measurement of the incorporation of amino acids into the protein. The above reaction solution in which synthesis reaction has stopped is passed through a gel filtration column that was previously equilibrated by a supply fluid having a similar composition to the external dialysis solution described in the above dialysis method. By reheating this filtrate solution to a suitable reaction temperature, synthesis reaction re-starts and protein synthesis progresses over several hours. The reaction and gel filtration operations are then repeated. A reaction temperature and time can be appropriately selected in accordance with the protein synthesis system used, and in a system using wheat embryo extract the gel filtration is preferably repeated every approximately 1 hour at 26 °C.

When bonding a labeling substance to the single chain antibody of this invention in the presence of a specific enzyme according to this kind of cell-free protein translation, the above-described translation reaction is conducted in the presence of the labeling substance and an enzyme that is capable of binding the labeling substance to a polypeptide of a linker part. More specifically, when bonding biotin as a labeling substance to a linker, a translation reaction is conducted in the presence of, for example, a biotin ligase (Avidity, manufactured by LLC, or the like) that is an enzyme that bonds biotin by recognizing an amino acid recognized by a biotin ligase that is previously inserted into a linker. An added amount of biotin and the biotin ligase is preferably an amount described in the instructions accompanying a commercially available product (enzyme).

When bonding labeling substances after synthesis of the protein, atter completing the translation reaction bonding may be conducted to a linker part of the single chain antibody in the translation reaction solution by a method suitable for the respective labeling substances, or bonding may be conducted by a method suitable for the respective labeling substances after purifying the single chain antibody by the method described below.

The thus obtained single chain antibody or labeled single chain antibody of this invention can be confirmed by a known method. More specifically, for example, a method involving measuring incorporation of amino acid into protein, separation by SDS-polyacrylamide gel electrophoresis and staining by Coomassie brilliant blue (CBB), or autoradiography (Endo, Y., et al., J. Biotech., 25, 221-230 (1992); Proc. Natl. Acad. Sci. USA, 97, 559-564 (2000)) or the like can be used.

Further, since the single chain antibody or labeled single chain antibody of interest is contained at a high concentration in the thus-obtained reaction solution, the single chain antibody or labeled single chain antibody of interest can be easily acquired from the reaction solution by a known method of separation and purification, such as dialysis, ion-exchange chromatography, affinity chromatography or gel filtration.

### (3) Utilization of labeled single chain antibody

The labeled single chain antibody of this invention can be used in a method for analyzing an antigen-antibody reaction by analyzing the binding ability thereof against an antigen. A method for analyzing an antigen-antibody reaction can be performed by comprising the following steps (I) to (VI):
(I) preparing a labeled single chain antibody under conditions in which a disulfide bond of a single chain antibody is retained, comprising the step of the following (1) or (2):
   (1) producing a labeled single chain antibody by subjecting a DNA, in which DNAs encoding a heavy chain and a light chain of an antibody having binding ability with a specific antigen are linked through a DNA encoding a linker comprising a nucleotide sequence that is capable of binding with a labeling substance in the presence of a specific enzyme after translation, to transcription and translation using a wheat cell-free protein synthesis system in the presence of a specific enzyme; or
   (2) producing a labeled single chain antibody by subjecting a DNA, in which DNAs encoding a heavy chain and a light chain that are variable regions of an antibody having binding ability with a specific antigen are linked through a DNA encoding a linker comprising a nucleotide sequence that is capable of binding with a labeling substance in the presence of a specific enzyme after translation, to transcription and translation using a wheat cell-free protein synthesis system in the presence of a specific enzyme;
(II) preparing a substance (adapter substance) that binds specifically with a labeling substance of a labeled single chain antibody in a case where the labeling substance of the labeled single chain antibody is an immobilizing substance, comprising the steps of:
   (1) immobilizing a substance (adapter substance) that binds specifically with a labeling substance of a labeled single chain antibody on a reaction plate compartmentalized into a plurality of regions;
   (2) removing the substance (adapter substance) that binds specifically with a labeling substance of a labeled single chain antibody that was not immobilized to the reaction plate in the preceding (i); and
   (3) before and after the step of the preceding (i) or (ii), removing nonspecific adsorption from the reaction plate as appropriate;
(III) preparing an immobilized single chain antibody in a case where a labeling substance is an immobilizing substance, comprising the steps of:
   (1) adding a required amount of the labeled single chain antibody prepared in (1) or (2) of the above (I) to a reaction plate compartmentalized into a plurality of regions having a substance (adapter substance) that binds specifically with the labeled single chain antibody of the above (II) on the surface thereof, to contact the labeled single chain antibody with the adapter substance;
   (2) removing a labeled single chain antibody that was not immobilized to the substance (adapter substance) that binds specifically to the labeled single chain antibody on the reaction plate in the preceding (1); and
   (3) following the preceding step (2), removing nonspecific adsorption from the reaction plate as appropriate;
(IV) preparing a labeled single chain antibody in a case where a labeling substance is a signal substance, comprising the steps of:
   (1) removing nonspecific adsorption from the reaction plate compartmentalized into a plurality of regions as appropriate; and
   (2) adding a required amount of the labeling substance of the labeled single chain antibody prepared in (1) or (2) of the above (I) to the reaction plate;
(V) adding a required amount of a test substance to each reaction plate according to the above (III) or (IV), and analyzing the binding ability of the labeled single chain antibody with the test substance; and
(VI) based on the binding ability result obtained in the preceding (V), qualitatively or quantitatively determining the interaction between the labeled single chain antibody and the test substance.

In the above antigen-antibody analysis method, conditions whereby a disulfide bond of a single chain antibody is retained are not particularly limited, as long as the conditions enable the retention of a disulfide bond of a labeled single chain antibody produced in a step of producing a labeled single chain antibody. More specifically, the method described in (iii) production of single chain antibody, which can be carried out by adjusting the concentration of a reducing agent in a translation reaction solution may be mentioned. Further, a method for removing an adapter substance and labeled single chain antibody comprises removing the adapter substance and labeled single chain antibody from the top of a reaction plate by washing the reaction plate several times using a washing buffer normally used by those skilled in the art. A method for removing nonspecific adsorption from an adapter substance immobilized on a reaction plate refers to using a blocking solution or the like that is conventionally used by those skilled in the art to fill the reaction plate. Thereafter, washing can be conducted several times with a buffer solution.

When a single chain antibody was immobilized, a method that is known in the art can be used to reduce nonspecific adsorption. Specific examples thereof include a method of precoating an array solid support using bovine serum albumin (BSA), reduced low fat milk, salmon sperm DNA, pig (mucosal) heparin or the like (Ausubel et al., Short Protocols in Molecular Biology, 3rd edition (1995)).

As a reaction plate used in the aforementioned antigen-antibody analysis method, a reaction plate that is suitable for an apparatus or method for analyzing an antigen-antibody reaction can be used. More specifically, when conducting analysis by enzyme-linked immunosorbent assay (ELISA) (Crowthjer, J. R., Methods in Molecular Biology, 42, (1995)), a plastic microtiter plate that is normally used in the ELISA method is preferred. When using a surface plasmon resonance method (Cullen, D. C. , et al., Biosciences, 3(4), 211-225 (1987-88)), a reaction plate in which a metallic thin film of gold, silver, platinum or the like is formed on a transparent reaction plate made of glass or the like is preferred. Further, when using molecule imaging using an evanescent field (Funatsu, T., et al., Nature, 374, 555-559 (1995)), a transparent medium made of glass or the like is preferable, and more preferably a reaction plate made of quartz glass is used. When using fluorescent imaging analysis, a nylon membrane or nitrocellulose membrane that is normally used for immobilizing a protein or the like can be used, and a plastic microtiter plate or the like can also be used. Further, a complex carbohydrate (for example, agarose and sepharose), acrylic resin (for example, polyacrylamide and latex beads), magnetic beads, silicon wafer and the like can also be used as reaction plates.

Bonding of an adapter substance to this kind of reaction plate can be performed according to a known method that is conventionally used in the art. More specifically, a diazo process, a peptide process (using acid amide derivatives, carboxychloride resin, maleic anhydride derivatives, isocyanate derivatives, cyanogen bromide activated polysaccharides, cellulose carbonate derivatives or the like), alkylation process, a method using a crosslinking reagent, a method using Ugi reaction and the like may be mentioned. When using a reaction plate made of glass or the like, a method that conducts physical adsorption can also be used. Further, a commercially available product such as streptavidin magnetic beads (manufactured by Promega Corp.) can also be used.

By bringing the thus obtained labeled single chain antibody into contact with a solution containing one or more test substances such as known antigens and analyzing the antigen-antibody reaction, it is possible to identify an antibody having binding specificity with respect to the antigen. The antigen may be a protein, or may be an organic compound, carbohydrate, nucleic acid or the like. These may be isolated, or may be recombinant or naturally occurring substances. The amount of an antigen used herein is preferably in the range of approximately 1 to 100 ng/µl. The time required for an antigen-antibody reaction is normally within the range of 5 minutes to 24 hours, and in general a time between 0.5 to 2 hours is preferable.

After the antigen-antibody reaction, in the case of an immobilized single chain antibody, a step can be added of washing the solid phase to which the antibody is bound using a buffer containing surfactants or the like that can be used biochemically. The composition of the buffer and the number of washings and the like can be appropriately selected in accordance with the strength of the antigen-antibody reaction and the like.

Further, in the aforementioned method for analyzing an antigen-antibody reaction, the antigen-antibody reaction can be analyzed by analyzing the binding ability between the immobilized single chain antibody and the antigen when the labeling substance is an immobilizing substance, and by analyzing the with the antigen in a solution when the labeling substance is a signal substance.

A method for quantitatively or qualitatively determining interaction between a labeled single chain antibody and a test substance can be conducted according to a known method that is conventionally used in the art. More specifically, a method such as ELISA, surface plasmon resonance, molecular imaging utilizing an evanescent field, fluorescent imaging analysis or a method using radioisotope labels may be mentioned.

A test substance such as an antigen may be any substance that may comprise an antigen. Specific examples thereof include body fluid such as blood, bacterial cell wall extract, and a protein mixture.

According to the method for analyzing an antigen-antibody reaction using the labeled single chain antibody of this invention and a reagent kit for measuring an antigen-antibody reaction comprising a reagent used in the analysis method, for example, a tool for diagnosing and analyzing the presence or absence of a human autoantibody, a cancer cell specific antigen and the like can be provided.

### Examples

This invention is described in further detail hereunder by means of examples, however the scope of this invention is not limited by these examples.

### Example 1. Preparation of biotinylated anti-Salmonella single chain antibody

### (1) Preparation of DNA encoding Salmonella single chain antibody and linker

An anti-Salmonella single chain antibody was selected as the single chain antibody of this invention to conduct the following test. The X-ray conformation of this antibody has already been analyzed, and molecular recognition with respect to sugar chain has been investigated in detail (Cygler, M., et al., Science, 253, 442-445 (1991); Bundle, D. R., et al., Biochemistry, 33, 5172-5182 (1994)). Lipopolysaccharide is present on the cell cortex of Salmonella bacteria, and anti-Salmonella antibody binds to O-antigen that is located at the most extracellular domain of the lipopolysaccharide (Anand, N. N., et al., Protein Engin., 3, 541-546 (1990)). It has been reported that a single chain antibody in which VL chain and VH chain, antigen-recognition sites that bind specifically to O-antigen, were connected by a specific linker was expressed in large quantities using *Escherichia coli* (Anand, N. N., et al., J. Biol. Chem., 266, 21874-21879 (1991)). Since a formation in which one disulfide bond is present respectively in the VL chain and VH chain is indispensable to synthesize a single chain antibody in an active form (Zdanov, A. L. Y., et al., Proc. Natl. Acad. Sci. USA, 91, 6423-6427 (1994)), the aforementioned single chain antibody was used as the subject of the method of this invention. DNA encoding anti-Salmonella single chain antibody was acquired by conducting a polymerase chain reaction (PCR) employing a plasmid containing DNA encoding a single chain antibody against wild-type Salmonella O-antigen (Anand, N. N., et al., J. Biol. Chem., 266, 21874-21879 (1991)) as a template and using primers comprising the nucleotide sequences represented by SEQ ID NOS: 2 and 3. The acquired DNA fragments were ligated into pGEMT-easy Vector (from Promega Corp.), and then digested with the restriction enzymes BgIII and NotI. The obtained DNA fragments were inserted into pEU vector that had been previously digested with the same restriction enzymes. PCR was conducted employing this plasmid as a template and using primers comprising the nucleotide sequences represented by SEQ ID NOS: 4 and 5 to introduce a stop codon. The thus produced plasmid was designated "scfv-pEU".

Next, DNA was produced in which a DNA sequence (SEQ ID NO: 1) encoding a biotin ligase recognition sequence was inserted in a linker part. First, PCR was carried out employing as a template the plasmid scfv-pEU produced as described above, and using LA Taq (manufactured by Takara Co., Ltd.) kit with primers comprising the nucleotide sequences represented by SEQ ID NOS: 6 and 7. The PCR reaction solution was prepared using 5 µl of 10x LA buffer, 5 µl of 25 mM magnesium chloride, 8 µl of 2.5 mM dNTP, 1 µl of 20 µM primer (for each primer), and 0.1 ng of template plasmid/50 µl, and reaction was conducted at 94 °C for 1 min × 1 cycle, 94 °C for 45 sec/ 55 °C for 1 min/ 72 °C for 1. 5 min × 30 cycles, and then 72 °C for 5 min. In accordance with a conventional method, the ends of amplified DNA fragments were blunted using KOD T4 polymerase (manufactured by NEB Inc.), the fragments were phosphorylated with Polynucleotide Kinase (NEB Inc.), and self-ligation was then carried out using Ligation High (manufactured by Toyobo Co., Ltd.) to produce a circular plasmid (FIG. 1; hereafter, this is sometimes referred to as "scFv-biotin-pEU").

### (2) Preparation of cell extract for weak reduced form of cell-free protein synthesis

Hokkaido-produced Chihoku wheat seeds (unsterilized) were added to a mill (Rotor Speed mill pulverisette 14 model, manufactured by Fritsch Inc.) at a rate of 100 g per minute, to gently pulverize the seeds at a rotation speed of 8,000 rpm. After sieving to collect fractions containing embryo having germinating capacity (mesh size 0.7 to 1.00 mm), a floating fraction containing embryo with germinating capacity was collected by flotation using a mixed solution of carbon tetrachloride and cyclohexane (volume ratio = carbon tetrachloride: cyclohexane = 2.4: 1), the organic solvent was removed by drying at room temperature, and impurities such as seed coat that were mixed therein were removed by blowing air at room temperature to obtain a coarse embryo fraction.

Next, embryo was selected from the coarse embryo fraction by utilizing difference in color using a belt type color sorter BLM-300K (manufactured by Anzai Manufacturing Co. Ltd.; selling agent: Anzai Corporation, Ltd.) in the manner described below. The color sorter is an apparatus having means to irradiate light on a coarse embryo fraction, means to detect reflected light and/or transmitted light from the coarse embryo fraction, means to compare detected values and reference values, and means to select and remove components with a detected value that is outside the reference values or components with a detected value that is within the range of reference values.

Coarse embryo fractions were supplied onto a beige color belt of the color sorter to form an amount of 1000 to 5000 grains/cm², light was irradiated by fluorescent lamp onto the coarse embryo fractions on the belt, and the reflected light was detected. The conveying speed of the belt was 50 m/min. A monochrome CCD line sensor (2048 pixels) was used as a light-receiving sensor.

First, in order to remove black-colored components (seed coat etc.) from the embryo, the reference value was set between the brightness of the embryo and the brightness of the seed coat, and components having a value outside the reference value were removed by suction. Subsequently, in order to screen for endosperm, the reference value was set between the brightness of the embryo and the brightness of endosperm, and components having a value outside the reference value were removed by suction. Suction was conducted using 30 suction nozzles provided at positions of about 1 cm apart on the upper part of the conveyor belt (the suction nozzles were arranged in a condition of 1 nozzle per 1 cm length).

By repeating this method, embryo was screened until the purity of the embryo (weight ratio of embryo contained per 1 g of arbitrary sample) was 98% or more.

The obtained wheat embryo fraction was suspended in distilled water with a temperature of 4 °C, and washed using an ultrasonic washer until the cleaning fluid lost its white turbidity. Next, the fraction was suspended in a solution containing 0.5 % Nonidet P40 (manufactured by Nacalai Tesque Inc.), and washed using an ultrasonic washer until the cleaning fluid lost its white turbidity to obtain wheat embryo, after which the following process was conducted at 4 °C.

Extracting solvent (80 mM HEPES-KOH (pH 7.8), 200 mM potassium acetate, 10 mM magnesium acetate, and 8 mM dithiothreitol (0. 6 mM each of 20 kinds of L-form amino acid may also be added)) of two-fold volume relative to the wet weight of the washed embryo was added thereto, and limited pulverization of the embryo was conducted 3 times for 30 seconds each time at 5,000 to 20,000 rpm using a Waring blender. Centrifuged supernatant obtained from this homogenate by centrifugation at 30, 000× g for 30 min using a high-speed centrifuge was centrifuged again under the same conditions to obtain supernatant. A decline in activity was not observed for this sample after long-term storage at -80 °C or below. The obtained supernatant was filtrated through a filter with a pore size of 0.2 µM (New SteraDisk 25; manufactured by Kurabo Industries Ltd.) for filter sterilization and removal of minute contaminants.

Next, this filtrate was subjected to gel filtration using a Sephadex G-25 column that was previously equilibrated with a mixed solution (40 mM HEPES-KOH (pH7.8), 100 mM potassium acetate, 5 mM magnesium acetate, and 0.3 mM each of 20 kinds of L-form amino acids (the amino acids may be omitted in accordance with the purpose of protein synthesis, or labeled amino acids may be used). After centrifuging the obtained filtrate again at 30,000× g for 30 min and adjusting the concentration of the collected supernatant so that A260 nm was 90 to 150 (A260/A280 = 1.4 to 1.6), the supernatant was stored at -80 °C or below until use in the protein synthesis reaction or dialysis process described hereunder.

### (3) Protein synthesis using weak reductive translation reaction solution (when adding biotin and biotin ligase at time of translation)

Transcription was conducted for the translation template DNA acquired in the above (1) using SP6 RNA polymerase (manufactured by Toyobo Co., Ltd.). The reaction solution contained 80 mM HEPES-KOH (pH 7.6), 16 mM magnesium acetate, 2 mM spermidine, 10 mM DTT, NTPs (2. 5 mM each), 0.8 U/µl RNase inhibitor, 50 µg/ml plasmid, and 1.2 U/µl SP6 RNA polymerase/ddw 400 µl. After incubation for 2 hours at 37 °C, purification was conducted by phenol/chloroform extraction and passage over a Nick column (manufactured by Amersham Pharmacia Inc.), followed by ethanol precipitation, and the ethanol precipitate was dissolved in 35 µl of purified water.

Translation reaction was conducted using the obtained mRNA. The translation reaction solution consisted of 1.2 mM ATP, 0.25 mM GTP, 15 mM creatine phosphate, 0.4 mM spermidine, 29 mM HEPES-KOH (pH 7.6), 95 mM potassium acetate, 2.7 mM magnesium acetate, 0.23 mM L-form amino acids, 0.58 U/µl RNase inhibitor (manufactured by Promega Corp.), 4nCi/µl 14C-Leu, 7.5 µg mRNA, 0.5 µM PDI, 19.5 µM biotin (Nacalai Tesque Inc.), 19.5 µg/µl biotin ligase (manufactured by Avidity Inc.), and 12 µl wheat embryo extract. Reaction was conducted by batch method for 3 hours at 26 °C. A translation reaction to which biotin was not added was also conducted as a control.

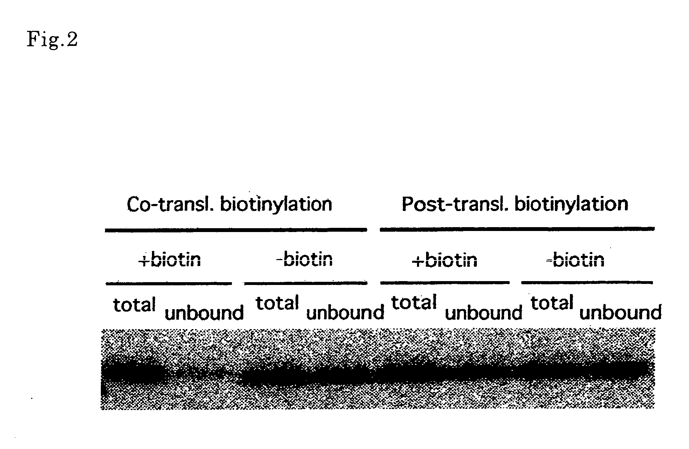

The reaction solution after 3 hours of translation reaction was centrifuged for 10 minutes at 15,000 rpm to separate solubilized components, and unreacted biotin remaining therein was removed using a G-25 spin column that was equilibrated with 50 mM Tris (pH 8.0). After diluting 20 µl of the spin column eluate with an equivalent amount of 50 mM Tris (pH 8.0) buffer solution, 5 µl of streptavidin magnetic beads (manufactured by Promega Corp.) was added, and this was mixed gently at room temperature. After collecting the magnetic beads using a magnetic field, supernatant fractions were acquired, and after separation by SDS-PAGE, the amount of anti-Salmonella single chain antibody was determined by autoradiography. The result is shown in co-transl. biotinylation of FIG. 2. As can be seen from the figure, when translation was conducted in the presence of biotin and a biotin ligase (in the figure: +biotin), the amount of antibody collected by magnetic beads through bonding with streptavidin was large, and conversely, when biotin was not added to the reaction solution (-biotin), almost no antibodies bound to the magnetic beads. It was thus clarified that biotin binds to anti-Salmonella single chain antibody according to the above-described method.

### (4) Protein synthesis using weak reductive translation reaction solution (when adding biotin and biotin ligase after translation reaction)

Biotin and a biotin ligase were added at 3 hours passage after the start of translation reaction, in a similar manner to the method of anti-Salmonella single chain antibody described in the above (1) to (3). The results are shown in post-transl. biotinylation of FIG. 2. As can be seen from the figure, it was found that for both the reaction solution to which biotin was added (+) and the reaction solution to which biotin was not added (-), almost no biotinylated antibody was removed with the magnetic beads. It was thus found that biotin ligase and biotin are preferably added during the translation reaction.

### Example 2. Immobilization of biotinylated single chain antibody and analysis of antigen-antibody reaction

### (1) Preparation of aldehyded Salmonella O-antigen

20 mg (2.8 µmol) of lipopolysaccharide (manufactured by Sigma Chemical Co., Ltd.) was dissolved in 20 µL of 0.25 M sodium hydroxide aqueous solution and stirred for 1 hour at 56 °C. After dialysis against distilled water, 200 mg (0.8 mmol) of sodium metaperfolate was added, and this mixture was stirred for 5 minutes while shading from light. After further adding 1 ml of ethylene glycol and stirring for 1 hour, the resulting mixture was subjected to dialysis against distilled water, and the dialysis residue was lyophilized to obtain powder of aldehyde-type Salmonella sugar chain. This powder was dissolved in 0.2 ml of 20 mM sodium borate buffer (pH 9.0) (10 mg/ml). After 3 times washing 0.1 ml of aminated magnetic beads (NH2-Mag; manufactured by Polyscience Inc.) with 0.4 ml of the same buffer to equilibrate, the beads were added to the above aldehyde-type Salmonella sugar chain solution, and reaction was conducted for 6 hours at room temperature. The magnetic beads were then washed 3 times with 0.4 ml of the same buffer. The ratio of immobilization of sugar chain onto the magnetic beads was obtained by determining the quantity of sugar chain remained in the supernatant by a phenol/sulfuric acid process. The binding ratio of sugar chain to the magnetic beads was 40% (0.13 µmol Salmonella sugar chain/ 100 µl magnetic beads).

### (2) Bonding of biotinylated anti-Salmonella single chain antibody and Salmonella sugar chain

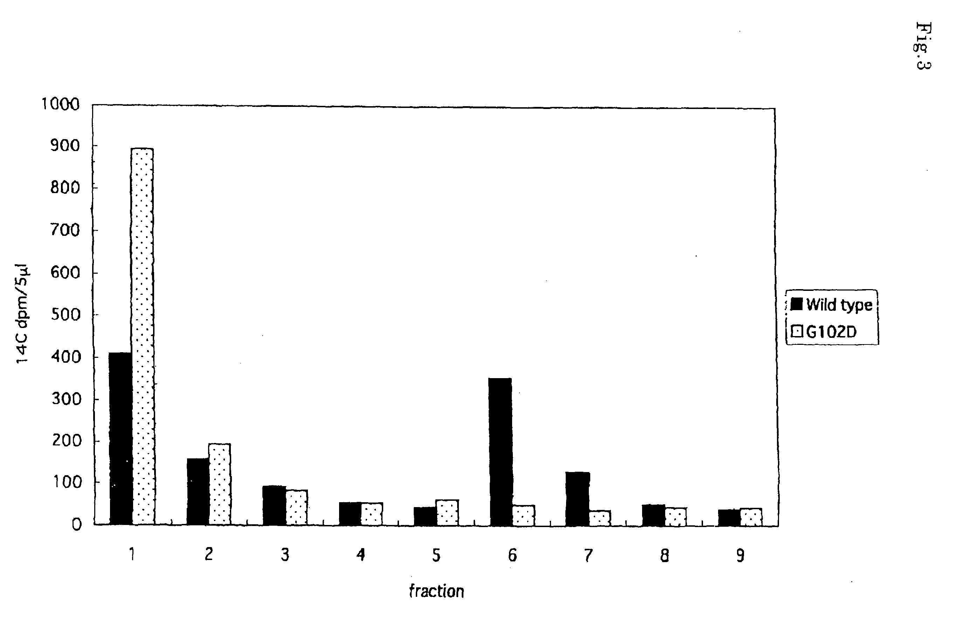

After synthesizing biotinylated anti-Salmonella single chain antibody by the method described in Example 1 (total 38 µl), excess biotin was removed by gel filtration with a G-25 spin column that was equilibrated with 10 mM PBST (pH8.0) and 0.6 mM CaCl₂. 40 µl of the protein solution was added onto a 96-well microplate together with 10 µl of immobilized Salmonella antigen (Sal-Mag) produced in the above (1) that had been previously washed with 25 µl of wheat embryo extract containing 0.6 mM CaCl₂. After gently mixing for 15 minutes, washing was performed 4 times with 40 µl of 0. 15 M NaCl/10 mM PBST (pH 8.0), and finally elution was conducted 4 times using an equivalent amount of 0.1 M glycine-HCL (pH 2.4). Single chain antibody that had not bound to the antigen was eluted by the initial washing, and single chain antibody that had bound to the antigen was eluted by the elution conducted thereafter. The amount of protein in each fraction (5 µl) was determined by 14 C count. The result is shown in FIG. 3. Here, for the purpose of confirming that the biotinylated single chain antibody retaine antigen specificity, the binding result for a case of biotinylating mutant G102D in a similar manner is also shown. As can be seen from the figure, while for the wild type close to 50 % of the total antibody was present in an active fraction (no. 6) eluted with a pH acidic solution, in contrast, for the mutant G102D an active fraction was completely non-existent and most of the antibody appeared in the pass-through fraction no. 1. This result shows that the biotinylated anti-Salmonella single chain antibody retained its original antigen binding activity, and that co-translational biotinylation progresses without any loss of antigen binding activity.

### (3) Measurement of dissociation equilibrium constant by Biomolecular Interaction Analysis System (Iasys)

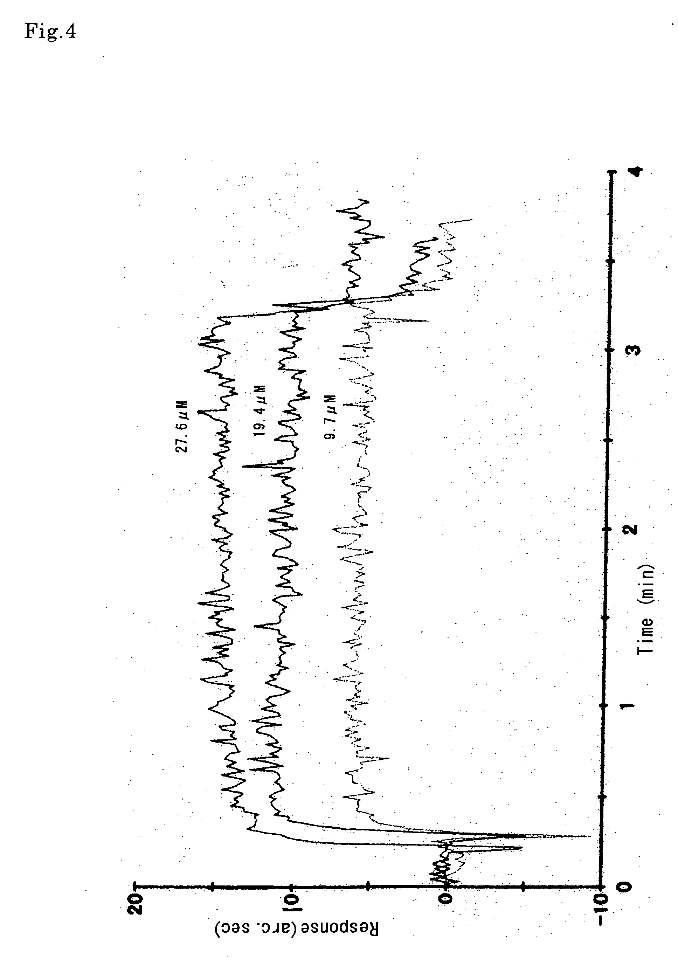

First, streptavidin (0.1 mg/ml; manufactured by Nacalai Tesque Inc.) was immobilized in a biotin cuvette (manufactured by Affinity Sensors Inc.) (immobilized amount: 674 arc sec., 27.2 ng, 0.97 pMol). Next, biotinylated single chain antibody prepared in Example 1 was purified using immobilized Salmonella sugar chain antigen (Sal-Mag) according to the method described in the above (2). 8.4 pmol/50 µl of purified biotinylated single chain antibody was obtained by being converted from a 14 C dpm value. This 50 µl amount was added to the above cuvette, and immobilized on the streptavidin (immobilized amount: 433.6 arc sec., 11.5 ng, 0.4 pmol). By adding thereto various concentrations (2.4, 4.8, 9.7, 12.9, 19.4 µM) of Salmonella sugar chain, association and dissociation curves were determined. FIG. 4 shows the results, while Table 1 lists the dissociation equilibrium constant obtained from the curves. Table 1 also lists values obtained for single chain antibody synthesized using viable cells of *Escherichia coli* by the same method for comparison (MacKenzie, C. R. et al., J. Biol. Chem., 271, 1527-1533 (1996)). As can be seen from the response curve of FIG. 4, it was possible to immobilize biotinylated single chain antibody onto streptavidin, and furthermore, a function for binding an antigen was retained. As shown in Table 1, when the dissociation equilibrium constant Kd was calculated on the basis of this curve, it was found that the constant was in the order of 1 × 10⁻⁷ to 10⁻⁸ M. Based on this result it was clarified that the single chain antibody prepared in Example 1 and immobilized by binding between biotin and streptavidin has a Kd value equivalent to that of complete anti-Salmonella antibody IgG.

**Table 1**

| antibody | **K**_{D} (**M**) | **K**_{diss} (**S**⁻¹) | **K**ₐₛₛ (**M**⁻¹ **S**⁻¹) s |
|---|---|---|---|
| cell-free system | 5.1×10 | 0.8×10 | 4.4×10 |
| in-vivo system | 6.5×10 | 3.1×10 | 1.8×10 |
| IgG | 1.4×10 | 1.2×10 | 8.7×10 |

### Comparative Example 1. Investigation of immobilization efficiency according to biotin binding position

### Addition of biotin to single chain antibody by chemical bonding

The method used in this example was in accordance with antibody labeling methods described in Immunobiochemical Methods, Biochemical Experiment Course, (Japanese Biochemical Society, Tokyo Kagaku Dojin (1986)).

By the method described in Example 1, a reaction solution was synthesized without adding biotin ligase and biotin at the time of translation reaction, and the solution was centrifuged at 15,000 rpm for 10 minutes to obtain supernatant. After diluting a 25 µl soluble fraction of supernatant with an equivalent amount of 1 M sodium bicarbonate solution, the buffer was exchanged using a G-25 Sephadex column, and 1 µl of NHS-biotin (N-hydroxysuccimide-biotin, 50 mg/ml DMSO) was then added. After reacting this solution over night at 4 °C, binding ability with an antigen was analyzed as described below. Analysis of binding activity with antigen

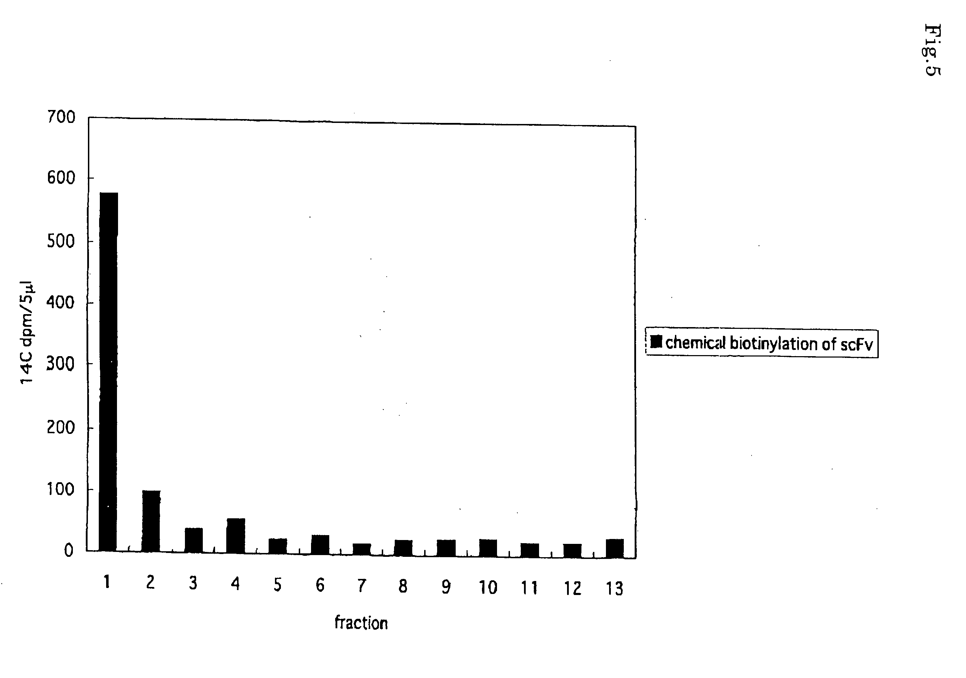

30 µl of reaction solution prepared in the above (1) was subjected to gel filtration with a G-25 spin column that was equilibrated with 10 mM of PBST (pH 8.0) and 0.6 mM of CaCl₂ to remove excess biotin. 40 µl of the protein solution was added onto a 96-well microplate together with 10 µl of immobilized Salmonella antigen (Sal-Mag) produced in Example 2 (1) that had been previously washed with 25 µl of wheat embryo extract containing 0.6 mM of CaCl₂. After gently mixing for 15 minutes, washing was conducted 4 times with 40 µl of 0.15 M NaCl/10 mM PBST (pH 8.0), and finally elution was conducted 4 times using an equivalent amount of 0.1 M glycine-HCL (pH 2.4). FIG. 5 shows the result. If the antibody retained its activity it would be eluted by the latter acidic buffer, however, as can be seen from the figure, the presence of protein was not observed in fraction numbers 10 to 13, and most of the protein was present in the first pass-through fraction. This result shows that the biotinylated single chain antibody produced by the aforementioned chemical process lost its antigen-binding activity.

### Example 3. Production of single chain antibody inserted with polyhistidine peptide and immobilization thereof

### (1) Production of single chain antibody containing polyhistidine peptide in a linker part

PCR was conducted employing scfv-pEU described in Example 1 (1) as a template, and using LA Taq (manufactured by Takara Co., Ltd.) kit with primers comprising the nucleotide sequences represented by SEQ ID NOS: 8 and 9. The PCR reaction solution was prepared with 5 µl of 10× LA buffer, 5 µl of 25 mM magnesium chloride, 8 µl of 2.5 mM dNTP, 1 µl of 20 µM primer (for each primer), and 0.1 ng of template plasmid/50 µl. The reaction was conducted by heating at 94 °C for 1 min × 1 cycle, 94 °C for 45 sec/ 55 °C for 1 min/ 72 °C for 1.5 min × 30 cycles, and then 72 °C for 5 min. In accordance with a conventional method, the ends of amplified DNA fragments were blunted using KOD T4 polymerase (manufactured by NEB Inc.), the fragments were phosphorylated with Polynucleotide Kinase (NEB Co., Ltd.), and self-ligation was then carried out using Ligation High (manufactured by Toyobo Co., Ltd.) to produce a circular plasmid (FIG. 1; hereafter, this is sometimes referred to as "scFv-pHIS-pEU").

After conducting transcription according to the method described in Example 1 (3) employing this plasmid as a template and purifying the mRNA, DTT in the translation reaction solution was replaced with 200 µM of mercaptoethanol to conduct a translation reaction. The reaction solution after 3 hours of translation reaction was centrifuged for 10 minutes at 15,000 rpm to separate solubilized components, and excess mercaptoethanol was removed using a G-25 spin column that was equilibrated with 50 mM phosphate buffer (pH 7.0), 500 mM NaCl, and 5% glycerol (binding buffer).

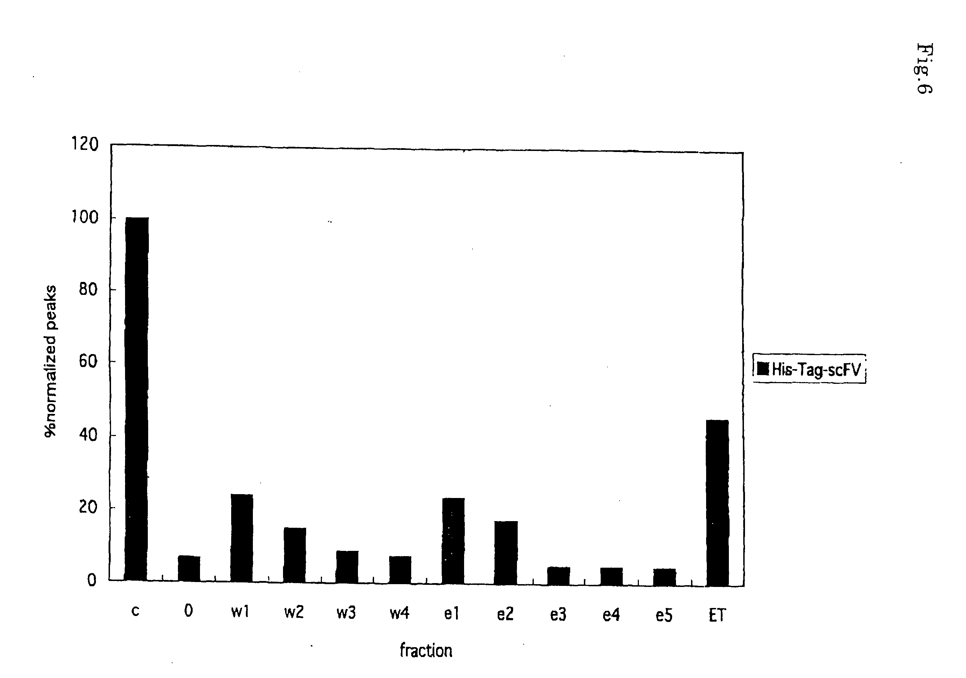

After diluting 20 µl of the spin column eluate with an equivalent amount of the binding buffer, 80 µl of the solution was passed over a 200 µl nickel column (50% resin) (metal affinity resin, Talon) that was previously washed 6 times with 150 µl of binding buffer, and this was incubated for one hour at room temperature. This column was washed 4 times (w1 to w4 in the figure) with 150 µl of 50 mM phosphate buffer (pH 7.0), 500 mM NaCl, 5% glycerol, and 6 mM imidazole (washing buffer), and elution was then carried out 5 times (e1 to e5 in the figure) with 150 µl of 50 mM phosphate buffer (pH 7.0), 500 mM NaCl, and 150 mM imidazole (elution buffer). The amount of single chain antibody contained in each fraction was measured by 14 C dpm value. FIG. 6 shows the result. In the figure, C indicates the 14 C dpm value in the total amount of protein-containing solution prior to passage over the column. The horizontal axis of the graph shows fraction numbers, w1 to w4 show the 14 C dpm values in fractions eluted by the washing buffer, while e1 to e5 show the 14 C dpm values in fractions eluted by the elution buffer. ET shows the total of the 14 C dpm values for e1 to e5.

Fraction numbers e1 to e5 indicate the existence of single chain antibody containing polyhistidine peptide binding specifically with nickel. As can be seen from the figure, it was found that approximately 50% of the total synthesized amount of single chain antibody could be purified by nickel column. It was confirmed by the method described in Example 2 that the purified single chain antibody also retained antigen-binding activity. This result indicates that a single chain antibody having polyhistidine peptide incorporated in a linker part thereof can be immobilized to a nickel solid phase in a condition in which it retains its activity.

### Industrial Applicability

According to the present invention, there is provided a single chain antibody or labeled single chain antibody that retains activity for binding specifically with an antigen. The single chain antibody can be bound to a solid phase via a labeling substance, and can be used to produce an antibody chip or the like. By synthesizing this single chain antibody using a cell-free protein translation system that allows an intramolecular disulfide bond to be retained, there can be provided an antibody having specific binding ability against an antigen that is higher than that of an antibody synthesized within a viable cell such as *Escherichia coli.*

## Claims

1. A single chain antibody comprising having a structure in which a heavy chain and a light chain of the antibody are crosslinked through a linker, or a labeled single chain antibody comprising carrying a labeling substance in a linker part of the single chain antibody.

2. A single chain antibody having a structure in which a heavy chain and a light chain of the antibody are crosslinked through a linker, or a labeled single chain antibody carrying a labeling substance in a linker part of the single chain antibody, wherein the heavy chain and the light chain of the antibody are variable regions.

3. A labeled single chain antibody having a structure in which a heavy chain and a light chain of an antibody are crosslinked through a linker, and carrying a labeling substance in the linker part, wherein the labeling substance is a substance that is capable of binding to a polypeptide of the linker part of the antibody in the presence of a specific enzyme.

4. A labeled single chain antibody having a structure in which a heavy chain and a light chain that are variable regions of the antibody are crosslinked through a linker, and carrying a labeling substance in the linker part, wherein the labeling substance is a substance that is capable of binding to a polypeptide of the linker part of the antibody in the presence of a specific enzyme.

5. A labeled single chain antibody having a structure in which a heavy chain and a light chain of an antibody are crosslinked through a linker, and carrying a labeling substance in the linker part, wherein the labeling substance is incorporated as one part of the linker part of the antibody.

6. A labeled single chain antibody having a structure in which a heavy chain and a light chain that are variable regions of the antibody are crosslinked through a linker, and carrying a labeling substance in the linker part, wherein the labeling substance is incorporated as one part of the linker part of the antibody.

7. A labeled single chain antibody having a structure in which a heavy chain and a light chain of the antibody are crosslinked through a linker, and carrying in the linker part a labeling substance that is capable of binding to a polypeptide of the linker part of the antibody in the presence of a specific enzyme, wherein the labeling substance is biotin and the enzyme is a biotin ligase.

8. A labeled single chain antibody having a structure in which a heavy chain and a light chain that are variable regions of the antibody are crosslinked through a linker, and carrying in the linker part a labeling substance that is capable of binding to a polypeptide of the linker part of the antibody in the presence of a specific enzyme, wherein the labeling substance is biotin and the enzyme is a biotin ligase.

9. The single chain antibody or labeled single chain antibody according to any one of claim 1 to 8, which has a Kd value that is equivalent to a Kd value of a naturally occurring antibody and which is produced by a cell-free protein translation system using wheat embryo.

10. A DNA, wherein DNAs encoding a heavy chain and a light chain of an antibody having binding ability against a specific antigen are linked through a DNA encoding a linker.

11. A DNA in which DNAs encoding a heavy chain and a light chain of an antibody having binding ability against a specific antigen are linked through a DNA encoding a linker, wherein the heavy chain and the light chain of the antibody are variable regions.

12. A DNA in which DNAs encoding a heavy chain and a light chain of an antibody having binding ability against a specific antigen are linked through a DNA encoding a linker, wherein the DNA encoding a linker comprises a nucleotide sequence that is capable of binding with a labeling substance in the presence of a specific enzyme after translation.

13. A DNA in which DNAs encoding a heavy chain and a light chain that are variable regions of an antibody having binding ability against a specific antigen are linked through a DNA encoding a linker, wherein the DNA encoding a linker comprises a nucleotide sequence that is capable of binding with a labeling substance in the presence of a specific enzyme after translation.

14. A DNA in which DNAs encoding a heavy chain and a light chain of an antibody having binding ability against a specific antigen are linked through a DNA encoding a linker that comprises a nucleotide sequence that is capable of binding with a labeling substance in the presence of a specific enzyme after translation, wherein the nucleotide sequence that is capable of binding with a labeling substance encodes an amino acid sequence that is recognized by a biotin ligase.

15. A DNA in which DNAs encoding a heavy chain and a light chain that are variable regions of an antibody having binding ability against a specific antigen are linked through a DNA encoding a linker that comprises a nucleotide sequence that is capable of binding with a labeling substance in the presence of a specific enzyme after translation, wherein the nucleotide sequence that is capable of binding with a labeling substance encodes an amino acid sequence which is recognized by a biotin ligase.

16. A method for producing a labeled single chain antibody, wherein the DNA according to any of claim 10 to 15 is subject to transcription and translation using a protein synthesis system in the presence of a labeling substance and a specific enzyme.

17. A method for producing a single chain antibody or a labeled single chain antibody, wherein the DNA according to claim 10 or 11 is subject to transcription and translation using a protein synthesis system.

18. The method for producing a single chain antibody or labeled single chain antibody according to claim 16 or 17, wherein the protein synthesis system is a wheat embryo-derived cell-free protein translation system, and a concentration of a reducing agent in a translation reaction solution thereof is a concentration whereby a disulfide bond of a single chain antibody to be produced is maintained and cell-free protein synthesis is enabled.

19. The method for producing a single chain antibody or a labeled single chain antibody according to claim 18, wherein the method is conducted in the presence of an enzyme that catalyzes a disulfide bond exchange reaction.

20. A single chain antibody or a labeled single chain antibody which has a Kd value that is equivalent to a Kd value of a naturally occurring antibody and is produced by the method for producing a single chain antibody or a labeled single chain antibody according to claim 19 using a wheat embryo-derived cell-free protein translation system.

21. A method for producing an immobilized single chain antibody, wherein any one of the antibodies described hereunder is brought into contact with a reaction plate compartmentalized into a plurality of regions having on the surface thereof a substance that binds specifically with a labeling substance of the antibody:
1) a labeled single chain antibody, wherein the antibody has a structure in which a heavy chain and a light chain of the antibody are crosslinked through a linker and the antibody carries a labeling substance in the linker part;
2) a labeled single chain antibody having a structure in which a heavy chain and a light chain of the antibody are crosslinked through a linker, and carrying a labeling substance in the linker part, wherein the heavy chain and the light chain of the antibody are variable regions;
3) a labeled single chain antibody having a structure in which a heavy chain and a light chain of the antibody are crosslinked through a linker, and carrying a labeling substance in the linker part, wherein the labeling substance is a substance that is capable of binding to a polypeptide of the linker part of the antibody in the presence of a specific enzyme;
4) a labeled single chain antibody having a structure in which a heavy chain and a light chain that are variable regions of the antibody are crosslinked through a linker, and carrying a labeling substance in the linker part, wherein the labeling substance is a substance that is capable of binding to a polypeptide of the linker part of the antibody in the presence of a specific enzyme;
5) a labeled single chain antibody having a structure in which a heavy chain and a light chain of the antibody are crosslinked through a linker, and carrying a labeling substance in the linker part, wherein the labeling substance is incorporated as one part of the linker part of the antibody;
6) a labeled single chain antibody having a structure in which a heavy chain and a light chain that are variable regions of the antibody are crosslinked through a linker, and carrying a labeling substance in the linker part, wherein the labeling substance is incorporated as one part of the linker part of the antibody;
7) a labeled single chain antibody having a structure in which a heavy chain and a light chain of the antibody are crosslinked through a linker, and carrying in the linker part a labeling substance that is capable of binding to a polypeptide of the linker part of the antibody in the presence of a specific enzyme, wherein the labeling substance is biotin and the enzyme is a biotin ligase;
8) a labeled single chain antibody having a structure in which a heavy chain and a light chain that are variable regions of the antibody are crosslinked through a linker, and carrying in the linker part a labeling substance that is capable of binding to a polypeptide of the linker part of the antibody in the presence of a specific enzyme, wherein the labeling substance is biotin and the enzyme is a biotin ligase.

22. The method for producing an immobilized single chain antibody of claim 21, wherein two or more kinds of different immobilized single chain antibodies are immobilized on a reaction plate compartmentalized into a plurality of regions.

23. The production method according to claim 21 or 22, wherein a labeling substance is biotin and a substance that binds specifically with the labeling substance is streptavidin.

24. An immobilized single chain antibody prepared by the production method according to any one of claim 21 to 23.

25. A method for analyzing an antigen-antibody reaction, wherein a test substance is brought into contact with the immobilized single chain antibody of claim 24, and binding ability of the test substance against the immobilized single chain antibody is analyzed.

26. A method for analyzing an antigen-antibody reaction, comprising the steps of:
(1) preparing a labeled single chain antibody under conditions in which a disulfide bond of a single chain antibody is retained, comprising the step of the following (i) or (ii):
(i) producing a labeled single chain antibody by subjecting a DNA, in which DNAs encoding a heavy chain and a light chain of an antibody having binding ability with a specific antigen are linked through a DNA encoding a linker comprising a nucleotide sequence that is capable of binding with a labeling substance in the presence of a specific enzyme after translation, to transcription and translation using a wheat cell-free protein synthesis system in the presence of a specific enzyme; or
(ii) producing a labeled single chain antibody by subjecting a DNA, in which DNAs encoding a heavy chain and a light chain that are variable regions of an antibody having binding ability with a specific antigen are linked through a DNA encoding a linker comprising a nucleotide sequence that is capable of binding with a labeling substance in the presence of a specific enzyme after translation, to transcription and translation using a wheat cell-free protein synthesis system in the presence of a specific enzyme;
(2) preparing a substance (adapter substance) that binds specifically with a labeling substance of a labeled single chain antibody in a case where the labeling substance of the labeled single chain antibody is an immobilizing substance, comprising the steps of:
(i) immobilizing a substance (adapter substance) that binds specifically with a labeling substance of a labeled single chain antibody to a reaction plate compartmentalized into a plurality of regions;
(ii) removing a substance (adapter substance) that binds specifically with a labeling substance of a labeled single chain antibody that was not immobilized to the reaction plate in the preceding (i); and
(iii) before and after the step of the preceding (i) or (ii), removing nonspecific adsorption from the reaction plate as appropriate;
(3) preparing an immobilized labeled single chain antibody in a case where a labeling substance of the labeled single chain antibody is an immobilizing substance, comprising the steps of:
(i) adding a required amount of the labeling substance of the labeled single chain antibody prepared in (i) or (ii) of the above (1) onto a reaction plate compartmentalized into a plurality of regions having a substance (adapter substance) of (2) that binds specifically with the labeling substance of the labeled single chain antibody on the surface thereof, whereby to contact;
(ii) removing a labeled single chain antibody that was not immobilized to the substance (adapter substance) that binds specifically to the labeled single chain antibody on the reaction plate in the preceding (i); and
(iii) following the preceding step (ii), removing nonspecific adsorption from the reaction plate as appropriate;
(4) preparing a labeled single chain antibody in a case where a labeling substance is a signal substance, comprising the steps of:
(i) removing nonspecific adsorption from a reaction plate compartmentalized into a plurality of regions as appropriate; and
(ii) adding a required amount of the labeling substance of the labeled single chain antibody prepared in (i) or (ii) of the above (1) onto the reaction plate;
(5) adding a required amount of a test substance onto each reaction plate according to the above (3) or (4), and analyzing the binding ability of a labeled single chain antibody with the test substance; and
(6) based on the binding ability result obtained in the above (5), qualitatively or quantitatively determining the interaction between the labeled single chain antibody and the test substance.

27. A reagent kit for measuring an antigen-antibody reaction, comprising a reagent to be used in the analysis method according to claim 25 or 26.


SEQUENCE LISTING

<110> CellFree Sciences Co., Ltd.

<120> SINGLE CHAIN ANTIBODY AND USE THEREOF

<130> ENDO-006-EP

<150> JP P 2002-210067

<151> 2002-07-18

<150> PCT/JP2003/009140

<151> 2003-07-18

<160> 9

<210> 1

<211> 36

<212> DNA

<213> Artificial Sequence

<220>

<223> Description of Artificial Sequence: Synthetic DNA

<400> 1

ggtttaaatg atatttttga agctcaaaaa attgaatggc atgaa 45

<210> 2

<211> 36

<212> DNA

<213> Artificial Sequence

<220>

<223> Description of Artificial Sequence: Synthetic DNA

<400> 2

ctaccagatc tgccatgcag atcgttgtta cccagg 36

<210> 3
<211> 30
<212> DNA
<213> Artificial Sequence
<220>
<223> Description of Artificial Sequence: Synthetic DNA
<400> 3
gcttgggccc agagctcacg gtcaggctcg 30

<210> 4
<211> 25
<212> DNA
<213> Artificial Sequence
<220>
<223> Description of Artificial Sequence: Synthetic DNA
<400> 4
ggctaagagc tcacggtcag gctcg 25

<210> 5
<211> 22
<212> DNA
<213> Artificial Sequence
<220>
<223> Description of Artificial Sequence: Synthetic DNA
<400> 5

gcctgcagct ggcgccatcg at 22

<210>6

<211> 36

<212> DNA

<213> Artificial Sequence

<220>

<223> Description of Artificial Sequence: Synthetic DNA

<400>6

caaaaaattg aatggcatga accgccgagc [?]caaac 36

<210>7

<211> 39

<212> DNA

<213> Artificial Sequence

<220>

<223> Description of Artificial Sequence: Synthetic DNA

<400> 7

agcttcaaaa atatcattta aacccgacgg gctgctttt 39

<210>8

<211> 30

<212> DNA

<213> Artificial Sequence

<220>

<223> Description of Artificial Sequence: Synthetic DNA

<400> 8

catcaccatc accatcaccc gccgagctcc 30

<210>9

<211> 16

<212> DNA

<213> Artificial Sequence

<220>

<223> Description of Artificial Sequence: Synthetic DNA

<400> 9

ggtaaccgac gggctg 16